# EUROPEAN PATENT SPECIFICATION

(11) **EP 1 096 954 B1**
(45) Date of publication and mention of the grant of the patent: **06.10.2004**
(21) Application number: 99935566.2
(22) Date of filing: 13.07.1999
(51) Int. Cl.: A61K 39/39, A61K 39/02, A61K 39/12, A61K 39/09, A61K 47/18, A61K 47/26, A61P 37/04

(54) **ADJUVANT AND VACCINE COMPOSITIONS CONTAINING MONOPHOSPHORYL LIPID A**
MONOPHOSPHORYLLIPID A ENTHALTENDE ADJUVANS- UND IMPFSTOFFZUSAMMENSETZUNGEN
COMPOSITIONS D'ADJUVANTS ET DE VACCINS CONTENANT UN LIPIDE MONOPHOSPHORYLE A

(30) Priority: 14.07.1998 US 115392
(43) Date of publication of application: 09.05.2001
(73) Proprietor: Wyeth Holdings Corporation, Madison, NJ 07940 (US)
(72) Inventor: LAPOSTA, Vincent, James, Pittsford, NY 14534 (US); ELDRIDGE, John, Hayward, Fairport, NY 14450 (US)
(74) Representative: Talbott, Dawn Jacqueline
(86) International application number: PCT/US1999/015942
(87) International publication number: WO 2000/003744

(56) References cited:
- EP-A- 0 812 593
- WO-A-98/01139
- FR-A- 2 672 496
- IVINS B ET AL: "Experimental anthrax vaccines: efficacy of adjuvants combined with protective antigen against an aerosol Bacillus anthracis spore challenge in guinea pigs" VACCINE, vol. 13, no. 18, 1995, pages 1779-1784, XP004057384
- FRIEDE M ET AL.: "Lyophilized liposomes as shelf items for the preparation of immunogenic liposome-petide conjugates" ANALYTICAL BIOCHEMISTRY, vol. 211, no. 1, 15 May 1993 (1993-05-15), pages 117-122, XP002129444

## Description

### BACKGROUND OF THE INVENTION

### 1. Field of the Invention

This invention relates to improved adjuvant and vaccine compositions, methods for preparing said improved adjuvant and vaccine compositions, and methods of using the improved compositions.

### 2. Description of the Prior Art

Conventional vaccines have been used for many years to protect humans and animals from a wide variety of infectious diseases. Typically, these conventional vaccines contain one or more antigens which may include an attenuated pathogen, killed pathogen, or an immunogenic component of a pathogen. In some vaccines, the antigen or antigens may be employed alone to elicit protective immune responses. In other vaccines, the antigen or antigens may be employed together with one or more adjuvants to enhance the immunogenicity of an antigen. One such adjuvant known to the art is monophosphoryl lipid A, which is derived from the lipopolysaccharide of Salmonella minnesota R595. It is also known to the art that monophosphoryl lipid A is a lipidic material which spontaneously aggregates with itself in an aqueous environment. Moreover, it is known that the degree of aggregation has an effect on the activity of monophosphoryl lipid A as an immunostimulant in that the aggregated monophosphoryl lipid A is less stimulatory.

WO 98 01139 A discloses a preparation for injection containing a lipid A analogue and a process for its preparation by dissolving a lipid A analogue in an alkaline aqueous solution and adding a buffer thereto.

EP A 0812593 discloses a vaccine composition comprising an antigen in conjunction with 3-O-deacylated monophosphoryl lipid A (MPL) wherein the particle size is small and in general does not exceed 120 nm when prepared.

Monophosphoryl lipid A is typically obtained as the triethylamine salt in the form of a lyophilized white powder. Being very hydrophobic, the lyophilized monophosphoryl lipid A does not readily form a clear solution when reconstituted with water but instead yields a turbid mixture with visible white particulates of heterogeneous size that settle out and further aggregate upon standing. To make an acceptable aqueous preparation of monophosphoryl lipid A, it is known to suspend the lyophilized monophosphoryl lipid A triethylamine salt at 1 to 2 mg/mL (w/v) in water containing 0.2% triethylamine, to heat the suspension at 65-70°C, and then to sonicate the mixture. The resulting aqueous preparation, slightly opalescent or clear, is an aqueous colloidal suspension. The triethylamine aids in the solubilization of the monophosphoryl lipid A and may be substituted with similar amounts of triethanolamine.

When aqueous preparations of monophosphoryl lipid A prepared as described hereinabove are frozen and then thawed, however, the monophosphoryl lipid A aggregates resulting in a turbid mixture quite similar in appearance to the turbid mixture of monophosphoryl lipid A prior to sonication. Similarly, when an aqueous preparation of monophosphoryl lipid A as described hereinabove is lyophilized and then rehydrated, the result is also a turbid mixture of aggregated monophosphoryl lipid A.

### SUMMARY OF THE INVENTION

The present invention provides to the art a lyophilized composition containing monophosphoryl lipid A, which composition exhibits an enhanced reconstitution feature and which avoids the settling out and aggregation problems of the prior art. In particular, the present invention provides a lyophilized composition comprising monophosphoryl lipid A, sugar and, optionally, an added amine based surfactant, and is capable of being reconstituted or rehydrated with an aqueous diluent to form, without further sonication, an aqueous colloidal suspension of monophosphoryl lipid A having a light transmission of at least 88%, as measured spectrophotometrically. The lyophilized composition according to the present invention comprises up to about 5 wt% monophosphoryl lipid A, greater than about 70 wt% sugar and from about 0 to about 30 wt% optionally added amine based surfactant, said wt% based on the total of the weights of monophosphoryl lipid A, sugar and, if present, amine based surfactant. Preferably, the lyophilized composition according to the present invention comprises up to about 5 wt% monophosphoryl lipid A, from about 70 to about 99.99 wt% sugar and from about 0 to about 28 wt% optionally added amine based surfactant. More preferably, the lyophilized composition according to the present invention comprises up to about 4 wt% monophosphoryl lipid A, from about 75 to about 99.99 wt% sugar and from about 0 to about 22 wt% optionally added amine based surfactant. The lyophilized composition may further comprise an immunologically effective amount of an antigen or antigens. The lyophilized composition of the present invention may be reconstituted or rehydrated with an aqueous diluent at concentrations up to about 210 mg of lyophilized composition per ml of aqueous diluent, preferably from about 10 mg of lyophilized composition per ml of aqueous diluent to about 210 mg of lyophilized composition per ml of aqueous diluent, to form, without further sonication, an aqueous colloidal suspension.

Another aspect of the present invention is a method of preparing an aqueous colloidal suspension of monophosphoryl lipid A in which the aqueous colloidal suspension is frozen for storage and then thawed for use without the problems of settling out and aggregation known in the prior art. By this method, monophosphoryl lipid A is mixed in an aqueous diluent and optionally with an amine based surfactant and also optionally an antigen or antigens. An aqueous colloidal suspension is formed by sonicating, optionally with heating, or other known methods, as described in greater detail hereinafter. Sugar, in an amount from about 10 mg/ml to about 200 mg/ml, is added to the mixture either before or after the formation of an aqueous colloidal suspension. The sugar may be in the form of a solid or in the form of an aqueous solution. The resulting aqueous colloidal suspension may then be frozen. Thawing the frozen aqueous colloidal suspension affords without further sonication an aqueous colloidal suspension containing monophosphoryl lipid A having a light transmission of greater than or equal to 88%, as measured spectrophotometrically. An antigen or antigens, as defined hereinafter, may be added to the thawed aqueous colloidal suspension to form a vaccine composition which may be administered to a vertebrate. Alternatively, if the aqueous colloidal suspension contains an antigen before freezing, the vaccine composition may be thawed and administered to a vertebrate.

The aqueous colloidal suspensions of the present invention are a special type of liquid suspension in which the particles of suspended monophosphoryl lipid A are present in very finely divided but not in dissolved form. The aqueous colloidal suspensions containing monophosphoryl lipid A, sugar and, optionally, an amine based surfactant according to the present invention are true suspensions not solutions, and do not have the property, unlike ordinary suspensions of monophosphoryl lipid A, of settling out and aggregation. The presence of the aqueous colloidal suspensions of the present invention can be determined by light transmission. Thus, an aqueous colloidal suspension containing monophosphoryl lipid A, sugar and optionally an amine based surfactant according to the present invention is one which exhibits a light transmission of greater than or equal to 88%, as measured spectrophotometrically.

The present invention solves the settling out and aggregation problems of the prior art, by providing the addition of sugar to an aqueous colloidal suspension of monophosphoryl lipid A prior to freezing or lyophilization. The sugar may be added either before or after formation of the aqueous colloidal suspension but must be added before freezing or lyophilization of the suspension. The addition of sugar to an aqueous colloidal suspension of monophosphoryl lipid A prior to freezing or lyophilization provides a composition which, after freezing can be thawed to afford an aqueous colloidal suspension without further sonication or, alternatively, after lyophilization, can be reconstituted with a suitable aqueous diluent and afford without further sonication an aqueous colloidal suspension as described hereinabove. Suitable sugars include the monosaccharides, dextrose, mannose, galactose and fructose as well as the disaccharides sucrose, lactose, isomaltose, maltose and trehalose. Mixtures of sugars, for example sucrose and dextrose, may also be employed. These sugars are all non toxic and pharmaceutically acceptable. Preferred are sucrose and dextrose. The sugar may be in the form of a solid or in the form of an aqueous solution. Suitable aqueous diluents include water or saline and can also include an antigen or antigens and, may additionally contain preservatives or additional adjuvants, or other pharmaceutically acceptable additives, vehicles, or carriers. Suitable amine based surfactants include triethylamine (TEA) and triethanolamine (TEM).

A further aspect of the invention is a reconstituted or rehydrated aqueous colloidal suspension which, despite the elimination of a further sonication step, is obtained upon reconstitution of the lyophilized composition described hereinabove with an aqueous diluent. As discussed hereinabove, before the present invention, a sonication step was necessary in order to obtain an aqueous colloidal suspension containing monophosphoryl lipid A. However, it has now been found that when an aqueous diluent is added to the lyophilized composition described hereinabove, an aqueous colloidal suspension containing monophosphoryl lipid A is obtained without further sonication. The reconstituted aqueous colloidal suspension so obtained exhibits a light transmission of greater than or equal to 88%, when measured spectrophotometrically. Surprisingly, the reconstituted aqueous colloidal suspension so obtained is capable of being frozen and, after thawing, again reforming an aqueous colloidal suspension which exhibits a light transmission of greater than or equal to 88%. The reconstituted aqueous colloidal suspension of the present invention comprises up to about 2.5 mg of monophosphoryl lipid A per ml of aqueous diluent, from about 10 to 200 mg of sugar per ml of aqueous diluent, and from about 0 to about 6 mg of amine based surfactant per ml of aqueous diluent. Preferably, the reconstituted aqueous colloidal suspension of the present invention comprises up to about 2.0 mg of monophosphoryl lipid A per ml of aqueous diluent, from about 20 to 150 mg of sugar per ml of aqueous diluent and from about 0 to about 3 mg of amine based surfactant per ml of diluent. The reconstituted aqueous colloidal suspension may further comprise an immunologically effective amount of an antigen or antigens. Suitable sugars, amine based surfactants and aqueous diluents are as described hereinabove.

A further aspect of the invention is a vaccine composition comprising the lyophilized composition and the reconstituted aqueous colloidal suspension described hereinabove in combination with an immunologically effective amount of an antigen or antigens. The effective amount of an antigen or antigens may be optionally provided in the aqueous diluent. In particular, the vaccine composition further comprises an immunologically effective amount of an antigen or antigens derived from or produced by a bacterium, a virus, a parasite, a cancer cell or an allergen. An effective amount of antigen is defined as that amount of antigen that when administered to an animal or a human evokes an immune response as measured by production of specific antibodies or cell-mediated effector mechanisms. Immunologically effective amounts of an antigen or antigens are in general from about 1µg or less to 5 mg. An effective amount of the monophosphoryl lipid A adjuvant is the amount of monophosphoryl lipid A that when added to a vaccine will enhance the magnitude or quality or duration of the immune response to the antigen or antigens in the vaccine. An effective amount of the adjuvant monophosphoryl lipid A is in the range of about 1 µg to about 1 mg.

Suitable antigens for the vaccine compositions of the present invention include any entity capable of producing an antibody or cell-mediated immunological response directed specifically against that entity in a vertebrate exposed to the antigen. One or more antigens may be employed. The antigen or antigens may be derived from pathogenic micro-organisms including viruses, bacteria, mycoplasmas, fungi, protozoa and other parasites. Further, the antigen or antigens may be derived from sources other than microorganisms, for example, cancer cells or allergens. The antigen or antigens may be all or part of a pathogenic microorganism, or all or part of a protein, glycoprotein, glycolipid, polysaccharide or lipopoly-saccharide which is associated with the organism, or the antigen or antigens may be a polypeptide or other entity which mimics all or part of such a protein, glycoprotein, glycolipid, polysaccharide or lipopolysaccharide.

Pathogenic microorganisms from which antigens may be produced or derived for vaccine purposes are well known in the field of infectious diseases, as listed in, for example, Medical Microbiology, Second Edition, (1990) J.C. Sherris (ed.), Elsevier Science Publishing Co., Inc., New York, and Zinsser Microbiology, 20th Edition (1992), W.K. Joklik et al. (eds.), Appleton & Lange Publishing Division of Prentice Hall, Englewood Cliffs, New Jersey. Examples of organisms of interest for human vaccines include *Chlamydia,* Nontypeable *Haemophilus influenzae*, *Helicobacter pylori*, *Moraxella catarrhalis, Neisseria gonorrhoeae, Neisseria meningitidis, Salmonella typhi*, *Streptococcus pneumoniae,* Group A *Streptococcus,* Group B Streptococcus, Herpes Simplex Virus, Human Immunodeficiency Virus, Human Papilloma Virus, Influenza, Measles, Parainfluenza, Respiratory Syncytial Virus, Rotavirus, Norwalk Virus, and others.

The antigen or antigens may include glycoconjugates which comprise polysaccharide antigen or antigens, for example, bacterial capsular polysaccharide or fragment thereof, chemically linked to a protein carrier molecule in order to enhance immunogenicity. Methods for preparing conjugates of bacterial capsular polysaccharide and protein carrier molecules are well known in the art and can be found, for example, in Dick and Burret, *Contrib Microbiol Immunol.* 10:48-114 (Cruse JM, Lewis RE Jr., eds; Basel Kruger (1989). Suitable conjugates, including pneumococcal glycoconjugate, are described in greater detail in U.S. 4,673,574, U.S. 4,761,283, U.S. 4,902,506, U.S. 5,097,020 and U.S. 5,360,897.

Also provided is a method of immunizing a vertebrate through vaccination which comprises administrating an effective amount of a vaccine composition according to the present invention to said vertebrate.

Also provided is a method for the preparation of a lyophilized composition comprising:
a. suspending monophosphoryl lipid A in an amount up to about 5 mg/ml and, optionally, an amine based surfactant in an amount from 0 to about 6 mg/ml in an aqueous diluent;
b. forming an aqueous colloidal suspension having a light transmission of greater than or equal to 88%, as measured spectrophotometrically;
c. adding sugar at about 10 to 200 mg/ml either before or after forming the aqueous colloidal suspension;
d. lyophilizing the sugar containing aqueous colloidal suspension; and
e. recovering a lyophilized composition.

Also provided is a method for preparing a lyophillized composition comprising:
a. heating lipopolysaccharide of gram negative bacteria Salmonella minnesota R595 in a mineral acid of moderate strength for a sufficient period of time to obtain a monophosphoryl derivative;
b. dissolving the monophosphoryl derivative in an organic solvent and drying;
c. treating the monophosphoryl derivative with mild alkali to remove a base labile fatty acid chain at the position to yield 3-deacylated monophosphoryl lipid A;
d. purifying the 3-deacylated monophosphoryl lipid A by liquid chromatography and recovering monophosphoryl lipid A;
e. suspending monophosphoryl lipid A in an amount up to about 5 mg/ml and, optionally, an amine based surfactant in an amount from 0 to about 6 mg/ml in an aqueous diluent;
f. forming an aqueous colloidal suspension having a light transmission of greater than or equal to 88%, as measured spectrophotometrically;
g. adding sugar at about 10 to 200 mg/ml either before or after forming the aqueous colloidal suspension;
h. lyophilizing the sugar containing aqueous colloidal suspension;and
i. recovering a lyophilized composition.

Also provided is a method for the preparation of an aqueous colloidal suspension containing monophosphoryl lipid A capable of being frozen and thawed comprising:
a. suspending monophosphoryl lipid A in an amount up to about 5 mg/ml and, optionally, an amine based surfactant in an amount from 0 to about 6 mg/ml in an aqueous diluent;
b. forming an aqueous colloidal suspension having a light transmission of greater than or equal to 88%, as measured spectrophotometrically;
c. adding sugar at about 10 to 200 mg/ml either before or after forming the aqueous colloidal suspension;
d. freezing the sugar containing aqueous colloidal suspension; and
e. thawing and recovering the aqueous colloidal suspension.

### DESCRIPTION OF THE PREFERRED EMBODIMENTS

The preparation of monophosphoryl lipid A is described in U.S. Patent No. 4,912,094. Briefly, monophosphoryl lipid A is produced by refluxing lipopolysaccharide (or lipid A) obtained from heptoseless mutants of gram negative bacteria, Salmonella minnesota R595, in mineral acid solutions of moderate strength (e.g., 0.1N HCl) for a period of approximately 30 minutes. Suitable mineral acids include hydrochloric, sulfuric and the like. This treatment results in the loss of the phosphate moiety at position 1 of the reducing-end glucosamine. The core carbohydrate is removed from the 6' position of the non-reducing glucosamine during this treatment. The result is a monophosphoryl derivative of lipid A. The monophosphoryl derivative of lipid A is dissolved in organic solvents and treated with very mild alkali which removes the base-labile fatty acid chain at the 3 position to yield 3-O-desacyl-4'-monophosphoryl lipid A, indicating that position 3 of the reducing end glucosamine is de-O-acylated. Chemically it is a mixture of 3-deacylated monophosphoryl lipid A with 4, 5 or 6 acylated chains. Suitable organic solvents include methanol (alcohols), dimethyl sulfoxide, dimethylformamide, chloroform, dichloromethane and the like as well as mixtures thereof. Combinations of water and one or more of these organic solvents also can be employed. Suitable alkaline bases can be chosen from among various hydroxides, carbonates, phosphates and amines. illustrative bases include the inorganic bases such as sodium hydroxide, potassium hydroxide, sodium carbonate, potassium carbonate, sodium bicarbonate, potassium bicarbonate, and the like, and organic bases such as alkyl amines and include, but are not limited to, diethylamine, triethylamine and the like. The 3-O-desacyl-4'-monophosophoryl lipid A is purified by liquid chromatography and converted to the monobasic triethylamine (triethylammonium) salt.

The term monophosphoryl lipid A as used herein means 3-O-desacyl-4'-monophosophoryl lipid A as the monobasic triethylamine (triethylammonium) salt.

To prepare the lyophilized composition of the present invention, the monophosphoryl lipid A is added to an aqueous diluent, preferably water, in amounts up to 5 mg of monophosphoryl lipid A per ml of aqueous diluent, preferably up to 2.5 mg/ml and more preferably from about 0.5 to 2.5 mg/ml. Optionally, an added amine based surfactant in an amount from about 0 to about 6 mg/ml, preferably 0 to 3 mg/ml is employed.

An aqueous colloidal suspension having a light transmission of greater than or equal to 88%, as measured spectrophotometrically is formed by sonication, optionally with heating, or other methods. Heating is optional but preferred to facilitate the formation of the aqueous colloidal suspension of monophosphoryl lipid A. Suitable sonication equipment include, for example, a probe sonicator (Vibracell VCX600; Sonica) attached to probes whose sizes are appropriate for the volume being processed or a bath sonicator such as the Model No. G112SP1T obtained from Laboratory Supplies Co. Inc., (Hicksville, NY). Other similar equipment used in the pharmaceutical industry would also be appropriate for sonication of monophosphoryl lipid A.
The aqueous colloidal suspension of monophosphoryl lipid A may be formed by methods other than sonication, for example, by shearing forces as would be generated in a microfluidizer.

Sugar is also added either before or after formation of the aqueous colloidal suspension, in amounts from 10 to 200 mg sugar per ml of aqueous diluent, preferably from about 20 to 150 mg/ml. The aqueous colloidal suspension, containing monophosphoryl lipid A, sugar and optionally an added amine based surfactant and optionally an immunologically effective amount of an antigen or antigens in the amounts recited hereinabove, is lyophilized to afford the lyophilized composition according to the present invention.

The aqueous colloidal suspension of monophosphoryl lipid A, sugar and, optionally, an amine based surfactant of an antigen or antigens is lyophilized to afford the lyophilized composition of the present invention. As is known to those skilled in the art, lyophilization is a process of drying in which water is sublimed from the product after it is frozen, by applying a vacuum. Specifics of lyophilizing or freeze-drying are described in Remington's Pharmaceutical Sciences. Chapter 84, page 1565, 18th Edition, A.R. Gennaro, Editor, 1990, Mack Publishing Company.

Whether an aqueous colloidal suspension is formed is determined by measuring the light transmission. It has been found that compositions having a light transmission of at least 88% exhibit the properties of colloidal suspensions. Light transmission is measured using a spectrophotometer in which is illuminated a liquid sample in a glass, quarts or plastic cuvette with a light path of 1 centimeter. The light may be in the visible or invisible spectrum, but for measurements of light transmission of this type a wavelength of 650 nm may appropriately be used. The amount of light passing through the sample (i.e. transmitted) is referenced to a blank cuvette containing the solvent or diluent in which the material is dissolved or suspended. Samples that do not absorb or scatter the light will exhibit 100% light transmission whereas those that absorb or scatter all the light will have 0% light transmission.

While not wishing to be bound by theory, it is believed that the advantageous results of the invention are obtained because the addition of sugar either before or after the formation of an aqueous colloidal suspension containing monophosphoryl lipid A prevents the monophosphoryl lipid A from aggregation either upon freezing or thawing of the aqueous colloidal suspension or upon lyophilizing the aqueous colloidal suspension and reconstitution or rehydration with an aqueous diluent. By including sugar in an aqueous colloidal suspension containing monophosphoryl lipid A prior to lyophilization, the lyophilized composition can be reconstituted with an aqueous diluent such as water or saline without the problem of reaggregation of the monophosphoryl lipid A. In addition, freezing of the reconstituted colloidal suspension or vaccine composition does not cause aggregation to reoccur. Similarly, by including sugar in an aqueous colloidal suspension containing monophosphoryl lipid A prior to freezing, upon thawing a frozen aqueous colloidal suspension is again obtained without the need for further sonication. The ability of sugar to prevent aggregation of the monophosphoryl lipid A is evident regardless of whether the aqueous colloidal suspension containing monophosphoryl lipid A is prepared in water alone, or in water containing triethylamine or triethanolamine.

Thus, the addition of sugar to monophosphoryl lipid A containing aqueous compositions, either before or after forming an aqueous colloidal suspension, provides surprising and unexpected results when such aqueous colloidal suspensions are either frozen and thawed or lyophilized and reconstituted. Such results further permit the advantageous preparation of vaccine compositions.

The following examples are provided to illustrate the invention.

### Example 1

### Preparation of Turbid Mixture and Measurement of Light Transmission

Monophosphoryl lipid A (RIBI ImmunoChem., Hamilton, MT) is suspended in water at 1 mg/ml (w/v) forming a turbid mixture with visible white particulates of heterogeneous size. The turbid mixture is placed in a Shimadzu UV-1601, UV-Visible Spectrophotometer and illuminated with light of 650 nm wavelength. The turbid mixture allows 3.3% of the incident light to pass (i.e. %transmission = 3.3). An aqueous colloidal suspension is not found.

### Example 2

### Preparation of Aqueous Colloidal Suspension and Measurement of Light Transmission

Monophosphoryl lipid A, at 1 mg/ml (w/v) is suspended in water containing 0.5% triethanolamine (v/v) (5.62 mg/mL (w/v)) or 0.2% triethylamine (v/v) (1.46 mglmL (w/v)). The samples are heated at 56-65°C for 10-15 minutes and sonicated using either a probe sonicator (Vibracell VCX600) set at 30% power using a tapered microtip or a bath sonicator (Model No. G112SP1T, Laboratory Supplies Co. Inc., Hicksville, NY.) used at full power for 2 to 3 minutes. A clear suspension is obtained and placed in a Shimadzu U-V-1601, UV-Visible Spectrophotometer and illuminated with light of 650 nm wavelength. The % light transmission is measured at ≥88%, indicating the formation of an aqueous colloidal suspension.

### Example 3

### Preparation of Aqueous Colloidal Suspension of Monophosphoryl lipid A and Lyophilization

Aqueous colloidal suspensions of monophosphoryl lipid A are formed by suspending monophosphoryl lipid A, at 1, 2 or 5 mg/mL (w/v) in water or water containing either 0.5% triethanolamine v/v (5.6 mg/mL w/v), or 0.2% triethylamine v/v (1.46 mg/mL w/v). Each Monophosphoryl lipid A suspension is heated for 10-15 minutes at 56°C to 65°C and then sonicated for a total of 2-3 minutes to obtain a clear suspension with no visual evidence of particulates. The samples (1 to 1.5 ml) are sonicated using either a probe sonicator (Vibracell VCX600) set at 30% power using a tapered microtip or a bath sonicator (Model No. G112SP1T, Laboratory Supplies Co. Inc., Hicksville, NY.) used at full power. Aliquots of the monophosphoryl lipid A aqueous colloidal suspensions above are diluted with an equal amount of water, or sucrose or dextrose solutions of varying concentrations. The resulting aqueous colloidal suspensions include monophosphoryl lipid A at 0.5, 1.0 or 2.5 mg/mL (w/v) and sucrose at final concentrations of 10, 50, 100 or 200 mg/ml (w/v) or dextrose at 10, 50, 100 or 170 mg/ml (w/v) as expressed in Table 1. The preparations contained either triethanolamine (TEM) at 2.81 or 5.62 mg/mL or triethylamine (TEA) at 0.73 mg/mL or no amine based surfactant. The samples are placed in a Shimadzu UV-1601, UV-Visible Spectrophotometer and illuminated with light of 650 nm wavelength. The % light transmission, as set forth in Table 1, ranges from 90.0 to 99.9%, indicating the formation of an aqueous colloidal suspension.

**TABLE 1.**

| COMPOSITION OF MONOPHOSPHORYL LIPID A FORMULATIONS | | | | | | |
|---|---|---|---|---|---|---|
| | | | | | | % light transmission |
| Sample | MPL mg/mL | sugar added | sugar mg/mL | Amine added | Added Amine mg/mL | |
| 1 | 0.5 | sucrose | 10 | TEM | 2.81 | 97.2 |
| 2 | 0.5 | dextrose | 10 | TEM | 2.81 | 97.1 |
| 3 | 0.5 | sucrose | 10 | TEM | 2.81 | 97.2 |
| 4 | 0.5 | dextrose | 10 | TEM | 2.81 | 97.3 |
| 5 | 0.5 | sucrose | 10 | TEA | 0.73 | 98.9 |
| 6 | 0.5 | sucrose | 10 | TEA | 0.73 | 98.4 |
| 7 | 0.5 | sucrose | 50 | TEM | 5.62 | 95.9 |
| 8 | 0.5 | sucrose | 50 | TEM | 5.62 | 96.0 |
| 9 | 0.5 | sucrose | 50 | TEM | 2.81 | 97.5 |
| 10 | 0.5 | dextrose | 50 | TEM | 2.81 | 97.4 |
| 11 | 0.5 | sucrose | 50 | TEM | 2.81 | 97.5 |
| 12 | 0.5 | dextrose | 50 | TEM | 2.81 | 97.4 |
| 13 | 0.5 | sucrose | 50 | TEA | 0.73 | 98.8 |
| 14 | 0.5 | sucrose | 50 | TEA | 0.73 | 99 |
| 15 | 0.5 | sucrose | 50 | - | 0 | 96.1 |
| 16 | 0.5 | sucrose | 50 | - | 0 | 96.0 |
| 17 | 0.5 | sucrose | 100 | TEM | 2.81 | 97.6 |
| 18 | 0.5 | dextrose | 100 | TEM | 2.81 | 97.6 |
| 19 | 0.5 | sucrose | 100 | TEM | 2.81 | 97.4 |
| 20 | 0.5 | dextrose | 100 | TEM | 2.81 | 97.6 |
| 21 | 0.5 | dextrose | 170 | TEM | 2.81 | 98.2 |
| 22 | 0.5 | dextrose | 170 | TEM | 2.81 | 98.1 |
| 23 | 0.5 | dextrose | 200 | TEM | 2.81 | 98.4 |
| 24 | 0.5 | sucrose | 200 | TEM | 2.81 | 98.4 |
| 25 | 0.5 | sucrose | 200 | TEM | 2.81 | 97.7 |
| 26 | 0.5 | sucrose | 200 | TEM | 2.81 | 97.8 |
| 27 | 0.5 | sucrose | 200 | TEA | 0.73 | 99.4 |
| 28 | 0.5 | sucrose | 200 | TEA | 0.73 | 99.4 |
| 29 | 0.5 | sucrose | 200 | TEA | 0.73 | 98.9 |
| 30 | 0.5 | sucrose | 200 | TEA | 0.73 | 98.9 |
| 31 | 1.0 | sucrose | 200 | TEM | 0.73 | 97.7 |
| 32 | 1.0 | sucrose | 200 | TEM | 0.73 | 97.6 |
| 33 | 1.0 | sucrose | 200 | TEM | 2.81 | 95.7 |
| 34 | 1.0 | sucrose | 200 | TEM | 2.81 | 95.6 |
| 35 | 2.5 | sucrose | 200 | TEM | 2.81 | 90.1 |
| 36 | 2.5 | sucrose | 200 | TEM | 2.81 | 90.0 |
| 37 | 2.5 | sucrose | 200 | TEA | 0.73 | 95.4 |
| 38 | 2.5 | sucrose | 200 | TEA | 0.73 | 95.3 |

### Lyophilization of Monophosphoryl Lipid A Adjuvant Compositions

The aqueous colloidal suspensions set forth in Table 1 are lyophilized by first freezing the samples in glass vials or polypropylene culture tubes on dry ice pellets for at least 30 minutes. They are then transferred to large freeze drying vessels (Labconco) and connected to a Virtus Freeze Dryer. The samples are lyophilized for 18 hours at a vacuum of 250 millitors and the condenser temperature of -50° C. The composition of the lyophilized adjuvant compositions are shown in Table 2.

### Reconstitution of Lyophilized Adjuvant Compositions

The lyophilized samples as set forth in Table 2 are reconstituted with either water or normal saline (0.9% NaCl w/v), the volume of which was equal to the volume of the aqueous colloidal suspension prior to lyophilization. Data showing the % light transmission of the samples after reconstitution with aqueous diluent are presented in Table 2. As shown in Table 2, the lyophilized compositions, containing sucrose or dextrose ranging from greater than 75% up to 99.4% of the composition by weight, gave rise to aqueous colloidal suspensions when rehydrated with water or saline. For samples 1-38 set forth in Table 2, the % transmission after rehydration ranged from 88.0% to 98.4% indicating the formation of an aqueous colloidal suspension. Samples 15 and 16, which contained 99% sugar by weight after lyophilization, were prepared without the addition of amines (triethylamine or triethanolamine) at the time of sonication. When rehydrated with either water or normal saline, %transmission values are measured at 96.1 and 93.6, respectively, indicating the formation of an aqueous colloidal suspension. These data show that when an aqueous colloidal suspension of Monophosphoryl lipid A prepared by sonication is lyophilized with an effective amount of sugar such as sucrose or dextrose it can be rehydrated with water or normal saline co regain an aqueous colloidal suspension.

**Table 2.**

| Light transmission properties of lyophilised Monophosphoryl Lipid A compositions after rehydration with water or normal saline. | | | | | |
|---|---|---|---|---|---|
| | Composition Wt.% after lyophilization | | | | % light transmission after rehydration |
| Samp le | % MPL | % sugar | % Added Amine | Diluent for rehydration | |
| 1 | 3.8 | 75.1 | 21.1 | water | 95.8 |
| 2 | 3.8 | 75.1 | 21.1 | water | 96.1 |
| 3 | 3.8 | 75.1 | 21.1 | saline | 95.5 |
| 4 | 3.8 | 75.1 | 21.1 | saline | 95.4 |
| 5 | 4.5 | 89.0 | 6.5 | water | 96.5 |
| 6 | 4.5 | 89.0 | 6.5 | saline | 94.8 |
| 7 | 0.9 | 89.1 | 10 | water | 95.7 |
| 8 | 0.9 | 89.1 | 10 | saline | 95.7 |
| 9 | 0.9 | 93.8 | 5.3 | water | 96.0 |
| 10 | 0.9 | 93.8 | 5.3 | water | 96.6 |
| 11 | 0.9 | 93.8 | 5.3 | saline | 95.7 |
| 12 | 0.9 | 93.8 | 5.3 | saline | 96.3 |
| 13 | 1.0 | 97.6 | 1.4 | water | 98.4 |
| 14 | 1.0 | 97.6 | 1.4 | saline | 96.3 |
| 15 | 1.0 | 99.0 | 0 | water | 96.1 |
| 16 | 1.0 | 99.0 | 0 | saline | 93.6 |
| 17 | 0.5 | 96.8 | 2.7 | water | 96.4 |
| 18 | 0.5 | 96.8 | 2.7 | water | 97.1 |
| 19 | 0.5 | 96.8 | 2.7 | saline | 95.8 |
| 20 | 0.5 | 96.8 | 2.7 | saline | 96.8 |
| 21 | 0.3 | 98.1 | 1.6 | water | 97.4 |
| 22 | 0.3 | 98.1 | 1.6 | saline | 96.6 |
| 23 | 0.2 | 98.4 | 1.4 | water | 97.7 |
| 24 | 0.2 | 98.4 | 1.4 | saline | 96.8 |
| 25 | 0.2 | 98.4 | 1.4 | water | 97.2 |
| 26 | 0.2 | 98.4 | 1.4 | saline | 96.9 |
| 27 | 0.2 | 99.4 | 0.4 | water | 98.4 |
| 28 | 0.2 | 99.4 | 0.4 | saline | 96.7 |
| 29 | 0.2 | 99.4 | 0.4 | water | 95.5 |
| 30 | 0.2 | 99.4 | 0.4 | saline | 96.7 |
| 31 | 0.5 | 99.1 | 0.4 | water | 97.1 |
| 32 | 0.5 | 99.1 | 0.4 | saline | 96.1 |
| 33 | 0.5 | 98.1 | 1.4 | water | 95.0 |
| 34 | 0.5 | 98.1 | 1.4 | saline | 94.6 |
| 35 | 1.2 | 97.4 | 1.4 | water | 89.8 |
| 36 | 1.2 | 97.4 | 1.4 | saline | 88.0 |
| 37 | 1.2 | 98.4 | 0.4 | water | 94.8 |
| 38 | 1.2 | 98.4 | 0.4 | saline | 90.8 |

### Example 4

Using the procedures set forth in Example 3, formulations containing monophosphoryl lipid A, sugar and amine in the amounts set forth in Table 3 are prepared. The light transmission of these formulations is measured and as set forth in Table 3, % light transmission ranges from 95.4 to 98.8% indicating the formation of an aqueous colloidal suspension.

**TABLE3.**

| COMPOSITION OF MONOPHOSPHORYL LIPID A FORMULATIONS | | | | | | |
|---|---|---|---|---|---|---|
| | | | | | | % Light Transmission |
| Sample | MPL mg/mL | sugar added | sugar mg/mL | Amine added | Added Amine mg/mL | |
| 39 | 0.5 | - | 0 | TEM | 5.62 | 95.8 |
| 40 | 0.5 | - | 0 | TEM | 2.81 | 97.1 |
| 41 | 0.5 | - | 0 | TEA | 0.73 | 98.8 |
| 42 | 0.5 | - | 0 | - | 0 | 95.8 |
| 43 | 0.5 | - | 0 | - | 0 | 95.9 |
| 44 | 0.5 | sucrose | 0.5 | TEM | 5.62 | 95.5 |
| 45 | 0.5 | sucrose | 1 | TEM | 5.62 | 95.4 |
| 46 | 0.5 | sucrose | 5 | TEM | 5.62 | 95.5 |
| 47 | 0.5 | sucrose | 10 | TEM | 5.62 | 95.6 |

Using the procedures set forth in Example 3, the formulations of Table 3 are lyophilized and reconstituted with water or saline as set forth in Table 4.

**Table 4.**

| Light transmission properties of lyophilized Monophosphoryl Lipid A formulations after rehydration with water or normal saline. | | | | | |
|---|---|---|---|---|---|
| | Wt. % of Composition after lyophilization | | | | % light transmission |
| Sample | % MPL | % sugar | % Added Amine | Diluent for rehydration | after rehydration |
| 39 | 8.2 | 0.0 | 91.8 | water | 58.6 |
| 40 | 15.1 | 0.0 | 84.9 | water | 58.2 |
| 41 | 40.7 | 0.0 | 59.3 | water | 22 |
| 42 | 100.0 | 0.0 | 0 | water | 32.8 |
| 43 | 100.0 | 0.0 | 0 | saline | 30.2 |
| 44 | 7.6 | 7.6 | 84.9 | water | 63.1 |
| 45 | 7.0 | 14.0 | 78.9 | water | 64.7 |
| 46 | 4.5 | 45.0 | 50.5 | water | 50.6 |
| 47 | 3.1 | 62.0 | 34.9 | water | 83.5 |

When samples lyophilized without sugars (samples 39-43) are rehydrated with water or saline the resultant preparation is turbid with suspended particulates. These samples exhibit a % transmission ranging from 22.0 to 58.6.

Similar results are obtained when samples 44-47 containing 7.6% to 62.0% sugar are rehydrated with water indicating that an aqueous colloidal suspension is not formed.

### Example 5

### Freezing and Thawing of Monophosphoryl Lipid A Sonicated in Aqueous Triethylamine in the Presence of Sucrose

Monophosphoryl lipid A is sonicated in water containing 0.2% triethylamine (v/v) and then admixed with an equal volume of water or with water containing added sucrose to yield a clear suspension containing monophosphoryl lipid A at 0.5 mg/mL (w/v) without sucrose or containing 100 mg/mL sucrose w/v and triethylamine at a final concentration of 0.1% v/v (0.73 mg/mL w/v). The samples (48 and 49) are placed in a Shimadzu UV-1601, UV-Visible Spectrophotometer and illuminated with light of 650 nm and each allowed 98.8% of the light to pass thus indicating the formation of an aqueous colloidal suspension. The colloidal suspensions are frozen and then thawed. Upon thawing, the monophosphoryl lipid A preparation without sucrose (sample 48) is turbid with particulates and has a % light transmission of 60.3% as measured in a Shimadzu UV-1601, UV-Visible Spectrophotometer and illuminated with light of 650 nm wavelength indicating that an aqueous colloidal suspension is not formed. The monophosphoryl lipid A containing sucrose (sample 49) remains clear after freezing and thawing and has a % light transmission of 97.8% as measured in a Shimadzu UV-1601, UV-Visible Spectrophotometer and illuminated with light of 650 nm wavelength indicating the formation of an aqueous colloidal suspension. These data are displayed in Table 5.

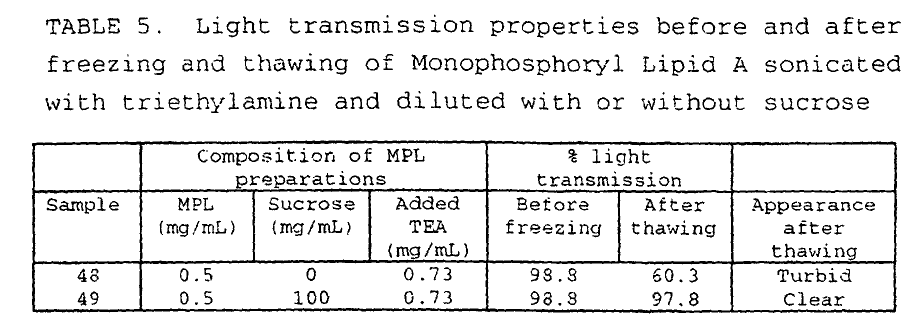

### Example 6 - Preparation of Vaccine Compositions:

### a. Preparation of aqueous colloidal suspensions of monophosphoryl lipid A

Using the procedures set forth in Example 3 above, a mixture of monophosphoryl lipid A in water of about 0.5 mg/ml and an amine-based surfactant triethanolamine at about 2.8 mg/ml is heated and sonicated to give an aqueous colloidal suspension. Either before or after sonication, but prior to freezing or lyophilizing, sucrose is added at a final concentration between about 10 to 200 mg/ml. The aqueous colloidal suspension so obtained may be either frozen and thawed for use in a vaccine composition or lyophilized and reconstituted with an aqueous diluent for use in a vaccine composition.

### b. Preparation of an aqueous vaccine composition from frozen monophosphoryl lipid A composition

The aqueous colloidal suspension of monophosphoryl lipid A, sucrose and triethanolamine prepared as in (a) above is frozen. It is then thawed and combined with an aqueous diluent containing an antigen, for example, a pneumococcal glycoconjugate prepared according to U.S. Patent 5,360,897, to obtain a vaccine composition containing up to about 400 micrograms monophosphoryl lipid A per ml and up to about 200 micrograms pneumococcal glycoconjugate per ml. To obtain a vaccine composition containing 400 micrograms of monophosphoryl lipid A and 200 micrograms of pneumococcal glycocongugate, for example, 0.8 ml of the thawed colloidal suspension may be combined with 200 micrograms of pneumococcal glycocongugate in 0.2 ml of water. This vaccine composition may then be administered to a vertebrate, preferably to a human, using about 0.1 to 1.0 ml per dose.

### c. Preparation of an aqueous vaccine composition from lyophilized monophosphoryl lipid A composition

The aqueous colloidal suspension of monophosphoryl lipid A, sucrose and triethanolamine prepared in (a) above is lyophilized. It is then reconstituted with an aqueous diluent containing an antigen, for example, a pneumococcal glyco-conjugate prepared according to U.S. Patent 5,360,897, to obtain a vaccine composition containing up to about 400 micrograms monophosphoryl lipid A per ml and up to about 200 micrograms pneumococcal glycoconjugate per ml. This vaccine composition may then be administered to a vertebrate, preferably to a human, using about 0.1 to 1.0 ml per dose.

### d. Preparation of a frozen aqueous vaccine composition

To the aqueous colloidal suspension of monophosphoryl lipid A, sucrose and triethanolamine prepared in (a) above is added an antigen, for example, a pneumococcal glycoconjugate prepared according to U.S. Patent 5,360,897 to obtain a vaccine composition. The vaccine composition is then frozen. The concentrations of monophosphoryl lipid A and pneumococcal glycoconjugate are adjusted by addition of a aqueous diluent, to up to about 400 micrograms per ml and up to about 200 micrograms per ml, respectively, either before freezing or after freezing and thawing, provided that the sucrose is kept at a concen-tration of about 10 to 200 mg/ml before freezing. The frozen and thawed vaccine composition may then be administered to a vertebrate, preferably to a human, using about 0.1 to 1.0 ml per dose.

### e. Preparation of a lyophilized vaccine composition

To the aqueous colloidal suspension of monophosphoryl lipid A, sucrose and triethanolamine prepared in (a) above is added, for example, a pneumococcal glycoconjugate prepared according to U.S. Patent 5,360,897 to obtain a vaccine composition. The antigen may be added either before or after the heating and sonicating steps. The amount of pneumococcal glycoconjugate added is calculated such that, upon subsequent reconstitution of the lyophilized vaccine composition, the aqueous mixture will contain up to about 400 micrograms of monophosphoryl lipid A per ml and up to about 200 micrograms pneumococcal glycoconjugate per ml. The vaccine composition is then lyophilized. Following lyophilization, the composition is reconstituted with an aqueous diluent. This reconstituted aqueous vaccine composition may then be administered to a vertebrate, preferably to a human, using about 0.1 to 1.0 ml per dose.

## Claims

1. A lyophilized composition comprising 3-O-desacyl-4'-monophosphoryl lipid A in an amount up to 5% by weight, greater than 70% by weight of sugar and 0 to 30% by weight of an added amine based surfactant.

2. A lyophilized composition as claimed in Claim 1 comprising 3-O-desacyl-4'-monophosphoryl lipid A in an amount up to 4% by weight, sugar from 75 to 99.99% by weight and amine based surfactant from 0 to 22% by weight.

3. A lyophilized composition as claimed in Claim 1 or Claim 2 wherein the sugar comprises dextrose, mannose, galactose, fructose, sucrose, lactose, isomaltose, maltose or trehalose.

4. A lyophilized composition as claimed in Claim 3 wherein the sugar is sucrose or dextrose.

5. A lyophilized composition as claimed in any one of Claims 1 to 4 wherein the amine based surfactant is triethylamine or triethanolamine.

6. A lyophilized composition as claimed in any one of Claims 1 to 5 further comprising an immunologically effective amount of an antigen or antigens.

7. A vaccine composition comprising an amount of a lyophilized composition as claimed in any one of Claims 1 to 6 and an immunologically effective amount of an antigen or antigens.

8. A vaccine composition as claimed in Claim 7 wherein the antigen or antigens are derived from or produced by a bacterium, a virus, a parasite, a cancer cell or an allergen.

9. A vaccine composition as claimed in Claim 7 wherein the antigen is a *Chlamydia,* Nontypeable *Haemophilus influenzae, Helicobacter pylori, Moraxella catarrhalis, Neisseria gonorrhoeae, Neisseria meningitidis, Salmonella typhi, Streptococcus* *pneumoniae,* Group A *Streptococci,* Group B *Streptococcus,* Herpes Simplex Virus, Human Immunodeficiency Virus, Human Papilloma Virus, Influenza, Measles, Parainfluenza, Respiratory Syncytial Virus, Rotavirus, or Norwalk Virus antigen.

10. A vaccine composition as claimed in any one of Claims 7 to 9 wherein the immunologically effective amount of an antigen or antigens is 1 µg to 5 mg.

11. A vaccine composition as claimed in any one of Claims 7 to 9 wherein an effective amount of 3-O-desacyl-4'-monophosphoryl lipid A is 1 µg to 1 mg.

12. A vaccine composition as claimed in any one of Claims 7 to 11 wherein the antigen is a conjugate comprising capsular polysaccharide of Streptococcus pneumoniae covalently attached to protein.

13. An aqueous composition comprising a lyophilized composition as claimed in any one of Claims 1 to 6 reconstituted with an aqueous diluent at an amount up to 210 mg of the lyophilized composition per ml of aqueous diluent, said aqueous composition in the form of an aqueous colloidal suspension having a light transmission of greater than or equal to 88% as measured spectrophotometrically.

14. An aqueous composition as claimed in Claim 13 comprising per ml of aqueous diluent up to 2.5 mg of 3-O-desacyl-4'-monophosphoryl lipid A from 10 to 200 mg of sugar and 0 to 6 mg of an optionally added amine based surfactant.

15. An aqueous composition as claimed in Claim 13 comprising per ml of aqueous diluent from .5 to 2.5 mg of 3-O-desacyl-4'-monophosphoryl lipid A, from 20 to 150 mg sugar, and from 0 to 3 mg of optionally added amine based surfactant.

16. An aqueous composition as claimed in any one of Claims 13 to 15 wherein said aqueous diluent comprises water or saline.

17. An aqueous composition as claimed in any one of Claims 13 to 16 wherein said aqueous diluent further comprises an antigen or antigens, an aluminum phosphate or other adjuvant, or a pharmaceutically acceptable carrier.

18. A vaccine composition comprising an amount of an aqueous composition as claimed any one of Claims 13 to 17 and an immunologically effective amount of an antigen or antigens.

19. A vaccine composition as claimed in Claim 18 wherein the antigen or antigens are derived from or produced by a bacterium, a virus, a parasite, a cancer cell or an allergen.

20. A vaccine composition as claimed in Claim 18 or Claim 19 wherein the antigen is a conjugate comprising capsular polysaccharide of Streptococcus pneumoniae covalently attached to protein.

21. A vaccine composition as claimed in any one of Claims 18 to 20 wherein the antigen is a *Chlamydia,* Nontypeable *Haemophilus influenzae, Helicobacter pylori, Moraxella catarrhalis, Neisseria gonorrhoeae, Neisseria meningitidis, Salmonella typhi, Streptococcus pneumoniae,* Group A *Streptococci,* Group B *Streptococcus,* Herpes Simplex Virus, Human Immunodeficiency Virus, Human Papilloma Virus, Influenza, Measles, Parainfluenza, Respiratory Syncytial Virus, Rotavirus, or Norwalk Virus antigen.

22. A vaccine composition as claimed in any one of Claims 18 to 21 wherein the effective amount of the antigen is 1 µg to 5 mg.

23. A vaccine composition as claimed in any one of Claims 18 to 21 wherein the effective amount of 3-O-desacyl-4'-monophosphoryl lipid A is 1 µg to 1 mg.

24. A method for the preparation of a lyophilized composition as defined in any one of claims 1 to 6 comprising:
a. suspending 3-O-desacyl-4'-monophosphoryl lipid A in an amount up to 5 mg/ml and, an amine based surfactant in an amount from 0 to 6 mg/ml in an aqueous diluent;
b. forming an aqueous colloidal suspension having a light transmission of greater than or equal to 88%, as measured spectrophotometrically;
c. adding sugar at 10 to 200 mg/ml either before or after forming the aqueous colloidal suspension;
d. lyophilizing the sugar containing aqueous colloidal suspension; and
e. recovering a lyophilized composition.

25. A method for preparing a lyophilized composition as defined in any one of claims 1 to 6 comprising:
a. heating lipopolysaccharide of gram negative bacteria Salmonella minnesota R595 in a mineral acid of moderate strength for a sufficient period of time to obtain a monophosphoryl derivative;
b. dissolving the monophosphoryl derivative in an organic solvent and drying;
c. treating the monophosphoryl derivative with mild alkali to remove a base labile fatty acid chain at the 3 position to yield 3-O-desacyl-4'-monophosphoryl lipid A;
d. purifying the 3-O-desacyl-4'-monophosphoryl lipid A by liquid chromatography and recovering monophosphoryl lipid A;
e. suspending 3-O-desacyl-4'-monophosphoryl lipid A in an amount up to 5 mg/ml and, an amine based surfactant in an amount from 0 to 6 mg/ml in an aqueous diluent;
f. forming an aqueous colloidal suspension having a light transmission of greater than or equal to 88%, as measured spectrophotometrically;
g. adding sugar at 10 to 200 mg/ml either before or after forming the aqueous colloidal suspension;
h. lyophilizing the sugar containing aqueous colloidal suspension; and
i. recovering a lyophilized composition.

26. A method for the preparation of an aqueous colloidal suspension as defined in any one of claims 13 to 17, containing 3-O-desacyl-4'-monophosphoryl lipid A capable of being frozen and thawed comprising:
a. suspending monophosphoryl lipid A in an amount up to 5 mg/ml and, an amine based surfactant in an amount from 0 to 6 mg/ml in an aqueous diluent;
b. forming an aqueous colloidal suspension having a light transmission of greater than or equal to 88%, as measured spectrophotometrically;
c. adding sugar at 10 to 200 mg/ml either before or after forming the aqueous colloidal suspension;
d. freezing the sugar containing aqueous colloidal suspension; and
e. thawing and recovering the aqueous colloidal suspension.

27. A method for the preparation of an aqueous colloidal suspension as defined in any one of claims 13 to 17, containing 3-O-desacyl-4'-monophosphoryl lipid A capable of being lyophilized and resuspended comprising:
a. suspending 3-O-desacyl-4'-monophosphoryl lipid A in an amount up to about 5 mg/ml and, an amine based surfactant in an amount from 0 to about 6 mg/ml in an aqueous diluent;
b. forming an aqueous colloidal suspension having a light transmission of greater than or equal to 88%, as measured spectrophotometrically;
c. adding sugar at about 10 to 200 mg/ml either before or after forming the aqueous colloidal suspension;
d. lyophilizing the sugar containing aqueous colloidal suspension; and
e. resuspending and reforming an aqueous colloidal suspension.

28. A method as claimed in Claim 26 or Claim 27 wherein said lyophilized aqueous colloidal suspension is combined with an aqueous diluent further containing an antigen or antigens, aluminum phosphate adjuvant or a pharmaceutically acceptable preservative, carrier or vehicle.

29. An aqueous colloidal suspension prepared according to the method of any one of Claims 26 to 28.

30. Use of lyophilized composition as claimed in any one of Claims 1 to 6 in the preparation of a vaccine.

31. Use of lyophilized composition as claimed in any one of Claims 1 to 6 in the preparation of an aqueous composition as claimed in any one of Claims 13 to 17.

32. Use of an aqueous composition as claimed any one of Claims 13 to 17 in the preparation of a vaccine.

33. Use of a vaccine composition as claimed in any one of Claims 7 to 12 or 18 to 23 in the preparation of a medicament for the immunization of a vertebrate.

## Patentansprüche

1. Gefriergetrocknete Zusammensetzung, welche 3-O-Desacyl-4'-monophosphoryl-Lipid-A in einer Menge bis zu 5 Gew.-%, mehr als 70 Gew.-% Zucker und 0 bis 30 Gew.-% eines zugegebenen, Amin-basierten Surfactanten umfasst.

2. Gefriergetrocknete Zusammensetzung wie in Anspruch 1 beansprucht, welche 3-O-Desacyl-4'-monophosphoryl-Lipid-A in einer Menge bis zu 4 Gew.-%, Zucker von 75 bis 99,99 Gew.-% und Amin-basierten Surfactanten von 0 bis 22 Gew.-% umfasst.

3. Gefriergetrocknete Zusammensetzung wie in Anspruch 1 oder Anspruch 2 beansprucht, wobei der Zucker Dextrose, Mannose, Galactose, Fructose, Sucrose, Lactose, Isomaltose, Maltose oder Trehalose umfasst.

4. Gefriergetrocknete Zusammensetzung wie in Anspruch 3 beansprucht, wobei der Zucker Sucrose oder Dextrose ist.

5. Gefriergetrocknete Zusammensetzung wie in einem der Ansprüche 1 bis 4 beansprucht, wobei der Amin-basierte Surfactant Triethylamin oder Triethanolamin ist.

6. Gefriergetrocknete Zusammensetzung wie in einem der Ansprüche 1 bis 5 beansprucht, welche ferner eine immunologisch wirksame Menge von einem Antigen oder Antigenen umfasst.

7. Impfstoffzusammensetzung, welche eine Menge einer gefriergetrockneten Zusammensetzung wie in einem der Ansprüche 1 bis 6 beansprucht und eine immunologisch wirksame Menge von einem Antigen oder Antigenen umfasst.

8. Impfstoffzusammensetzung wie in Anspruch 7 beansprucht, wobei das Antigen oder die Antigene von einem Bakterium, einem Virus, einem Parasiten, einer Krebszelle oder einem Allergen abgeleitet oder produziert werden.

9. Impfstoffzusammensetzung wie in Anspruch 7 beansprucht, wobei das Antigen ein *Chlamydia, Nontypeable Haemophilus influenzae, Helicobacter pylori, Moraxella catarrhalis, Neisseria gonorrhoeae, Neisseria meningitidis, Salmonella typhi, Streptococcus pneumoniae,* Gruppe A *Streptococci,* Gruppe B *Streptococcus,* Herpes simplex Virus, humanes Immunschwächevirus, humanes Papillomavirus, Influenza, Masern, Parainfluenza, Respiratory Syncytial Virus, Rotavirus oder Norwalk Virus Antigen ist.

10. Impfstoffzusammensetzung wie in einem der Ansprüche 7 bis 9 beansprucht, wobei die immunologisch wirksame Menge von einem Antigen oder Antigenen 1 µg bis 5 mg ist.

11. Impfstoffzusammensetzung wie in einem der Ansprüche 7 bis 9 beansprucht, wobei eine wirksame Menge von 3-O-Desacyl-4'-monophosphoryl-Lipid-A 1 µg bis 1 mg ist.

12. Impfstoffzusammensetzung wie in einem der Ansprüche 7 bis 11 beansprucht, wobei das Antigen ein Konjugat ist, welches kapselförmiges Polysaccharid von Streptococcus pneumoniae, kovalent an Protein gebunden, umfasst.

13. Wässerige Zusammensetzung, welche eine gefriergetrocknete Zusammensetzung wie in einem der Ansprüche 1 bis 6 beansprucht, rekonstituiert mit einem wässerigen Verdünnungsmittel bei einer Menge von bis zu 210 mg der gefriergetrockneten Zusammensetzung pro ml wässerigem Verdünnungsmittel umfasst, wobei besagte wässerige Zusammensetzung in Form einer wässerigen kolloidalen Suspension eine Lichtdurchlässigkeit größer als oder gleich 88% hat, wie spektrophotometrisch gemessen.

14. Wässerige Zusammensetzung wie in Anspruch 13 beansprucht, welche pro ml wässerigem Verdünnungsmittel bis zu 2,5 mg 3-O-Desacyl-4'-monophosphoryl-Lipid-A, von 10 bis 200 mg Zucker und 0 bis 6 mg eines gegebenenfalls zugegebenen Amin-basierten Surfactanten umfasst.

15. Wässerige Zusammensetzung wie in Anspruch 13 beansprucht, welche pro ml wässerigem Verdünnungsmittel von 0,5 bis 2,5 mg 3-O-Desacyl-4'-monophosphoryl-Lipid-A, von 20 bis 150 mg Zucker und von 0 bis 3 mg gegebenenfalls zugegebenen Amin-basierten Surfactanten umfasst.

16. Wässerige Zusammensetzung wie in einem der Ansprüche 13 bis 15 beansprucht, wobei besagtes wässeriges Verdünnungsmittel Wasser oder physiologische Kochsalzlösung umfasst.

17. Wässerige Zusammensetzung wie in einem der Ansprüche 13 bis 16 beansprucht, wobei besagtes wässeriges Verdünnungsmittel ferner ein Antigen oder Antigene, ein Aluminiumphosphat oder anderes Adjuvans oder einen pharmazeutisch annehmbaren Träger umfasst.

18. Impfstoffzusammensetzung, welche eine Menge einer wässerigen Zusammensetzung wie in einem der Ansprüche 13 bis 17 beansprucht und eine immunologisch wirksame Menge von einem Antigen oder Antigenen umfasst.

19. Impfstoffzusammensetzung wie in Anspruch 18 beansprucht, wobei das Antigen oder die Antigene durch ein Bakterium, ein Virus, einen Parasiten, eine Krebszelle oder ein Allergen abgeleitet oder produziert werden.

20. Impfstoffzusammensetzung wie in Anspruch 18 oder Anspruch 19 beansprucht, wobei das Antigen ein Konjugat ist, welches kapselförmiges Polysaccharid von Streptococcus pneumoniae, kovalent an Protein gebunden, umfasst.

21. Impfstoffzusammensetzung wie in einem der Ansprüche 18 bis 20 beansprucht, wobei das Antigen ein *Chlamydia, Nontypeable Haemophilus influenzae, Helicobacter pylori, Moraxella catarrhalis, Neisseria gonorrhoeae, Neisseria meningitidis, Salmonella typhi, Streptococcus pneumoniae,* Gruppe A *Streptococci,* Gruppe B *Streptococcus,* Herpes simplex Virus, humanes Immunschwächevirus, humanes Papillomavirus, Influenza, Masern, Parainfluenza, Respiratory Syncytial Virus, Rotavirus oder Norwalk Virus Antigen ist.

22. Impfstoffzusammensetzung wie in einem der Ansprüche 18 bis 21 beansprucht, wobei die wirksame Menge des Antigens 1 µg bis 5 mg beträgt.

23. Impfstoffzusammensetzung wie in einem der Ansprüche 18 bis 21 beansprucht, wobei die wirksame Menge von 3-O-Desacyl-4'-monophosphoryl-Lipid-A 1 µg bis 1 mg ist.

24. Verfahren zur Herstellung einer gefriergetrockneten Zusammensetzung wie in Ansprüchen 1-6 definiert, welches umfasst:
a. Suspendieren von 3-O-Desacyl-4'-monophosphoryl-Lipid-A in einer Menge bis zu 5 mg/ml und einen Amin-basierten Surfactanten in einer Menge von 0 bis 6 mg/ml in einem wässerigen Verdünnungsmittel;
b. Bilden einer wässerigen, kolloidalen Suspension mit einer Lichtdurchlässigkeit größer als oder gleich 88%, wie spektrophotometrisch gemessen;
c. Zugeben von Zucker bei 10 bis 200 mg/ml entweder vor oder nach Bilden der wässerigen, kolloidalen Suspension;
d. Gefriertrocknen der Zucker enthaltenden, wässerigen, kolloidalen Suspension; und
e. Gewinnen einer gefriergetrockneten Zusammensetzung.

25. Verfahren zur Herstellung einer gefriergetrockneten Zusammensetzung wie in Ansprüchen 1-6 definiert, welches umfasst:
a. Erhitzen von Lipopolysaccharid von gram-negativen Bakterien Salmonella minnesota R595 in einer Mineralsäure von moderater Stärke für einen ausreichenden Zeitraum, um ein Monophosphorylderivat zu erhalten;
b. Lösen des Monophosphorylderivats in einem organischen Lösungsmittel und Trocknen;
c. Behandeln des Monophosphorylderivats mit mildem Alkali, um eine Base-labile Fettsäurekette an der 3-Position zu entfernen, um 3-O-Desacyl-4'-monophosphoryl-Lipid-A zu erhalten;
d. Reinigen des 3-O-Desacyl-4'-monophosphoryl-Lipid-A durch Flüssigkeitschromatographie und Gewinnen von Monophosphoryl-Lipid-A;
e. Suspendieren von 3-O-Desacyl-4'-monophasphoryl-Lipid-A in einer Menge bis zu 5 mg/ml und einen Amin-basierten Surfactant in einer Menge von 0 bis 6 mg/ml in einem wässerigen Verdünnungsmittel;
f. Bilden einer wässerigen, kolloidalen Suspension mit einer Lichtdurchlässigkeit größer als oder gleich 88%, wie spektrophotometrisch gemessen;
g. Zugeben von Zucker bei 10 bis 200 mg/ml entweder vor oder nach Bilden der wässerigen, kolloidalen Suspension;
h. Gefriertrocknen der Zucker enthaltenden, wässerigen, kolloidalen Suspension; und
i. Gewinnen einer gefriergetrockneten Zusammensetzung.

26. Verfahren zur Herstellung einer wässerigen, kolloidalen Suspension wie in Ansprüchen 13-17 definiert, welche 3-O-Desacyl-4'-monophosphoryl-Lipid-A enthält, welche in der Lage ist, gefroren und getaut zu werden, welches umfasst:
a. Suspendieren von Monophosphoryl-Lipid-A in einer Menge bis zu 5 mg/ml und einen Amin-basierten Surfactanten in einer Menge von 0 bis 6 mg/ml in einem wässerigen Verdünnungsmittel;
b. Bilden einer wässerigen, kolloidalen Suspension mit einer Lichtdurchlässigkeit größer als oder gleich 88%, wie spektrophotometrisch gemessen;
c. Zugeben von Zucker bei 10 bis 200 mg/ml entweder vor oder nach Bilden der wässerigen, kolloidalen Suspension;
d. Gefrieren der Zucker enthaltenden, wässerigen, kolloidalen Suspension; und
e. Tauen und Rückgewinnen der wässerigen, kolloidalen Suspension.

27. Verfahren zur Herstellung einer wässerigen, kolloidalen suspension wie in Ansprüchen 13-17 definiert, welche 3-O-Desacyl-4'-monophosphoryl-Lipid-A enthält, welche in der Lage ist, gefriergetrocknet und resuspendiert zu werden, welches umfasst:
a Suspendieren von 3-O-Desacyl-4'-monophosphoryl-Lipid-A in einer Menge bis zu etwa 5 mg/ml und einen Amin-basierten Surfactanten in einer Menge von 0 bis etwa 6 mg/ml in einem wässerigen Verdünnungsmittel;
b. Bilden einer wässerigen, kolloidalen Suspension mit einer Lichtdurchlässigkeit größer als oder gleich 88%, wie spektrophotometrisch gemessen;
c. Zugeben von Zucker bei etwa 10 bis 200 mg/ml entweder vor oder nach Bilden der wässerigen, kolloidalen Suspension;
d. Gefriertrocknen der Zucker enthaltenden, wässerigen, kolloidalen Suspension; und
e. Resuspendieren und Umbilden einer wässerigen, kolloidalen Suspension.

28. Verfahren wie in Anspruch 26 oder Anspruch 27 beansprucht, wobei besagte gefriergetrocknete wässerige, kolloidale Suspension mit einem wässerigen Verdünnungsmittel vereinigt wird, welches ferner ein Antigen oder Antigene, Aluminiumphosphat-Adjuvans oder ein pharmazeutisch annehmbares Konservierungsmittel, Träger oder Vehikel enthält.

29. Wässerige, kolloidale Suspension, hergestellt gemäß dem Verfahren nach einem der Ansprüche 26 bis 28.

30. Verwendung von gefriergetrockneter Zusammensetzung wie in einem der Ansprüche 1 bis 6 beansprucht, bei der Herstellung eines Impfstoffs.

31. Verwendung von gefriergetrockneter Zusammensetzung wie in einem der Ansprüche 1 bis 6 beansprucht, bei der Herstellung einer wässerigen Zusammensetzung wie in einem der Ansprüche 13 bis 17 beansprucht.

32. Verwendung einer wässerigen Zusammensetzung wie in einem der Ansprüche 13 bis 17 beansprucht, bei der Herstellung eines Impfstoffs.

33. Verwendung einer Impfstoffzusammensetzung wie in einem der Ansprüche 7 bis 12 oder 18 bis 23 beansprucht, bei der Herstellung eines Medikaments zur Immunisierung eines Wirbeltiers.

## Revendications

1. Composition lyophilisée comprenant du 3-O-désacyl-4'-lipide monophosphoryle A en quantité jusqu'à 5% en poids, supérieure à 70% en poids de sucre et 0 à 30% en poids d'un tensio-actif à base d'amine ajouté.

2. Composition lyophilisée selon la revendication 1, comprenant du 3-O-désacyl-4'-lipide monophosphoryle A en quantité jusqu'à 4% en poids, 75 à 99,99% en poids de sucre et 0 à 22% en poids d'un tensio-actif à base d'aminé.

3. Composition lyophilisée selon la revendication 1 ou la revendication 2, dans laquelle le sucre comprend le dextrose, le mannose, le galactose, le fructose, le saccharose, le lactose, l'isomaltose, le maltose ou le tréhalose.

4. Composition lyophilisée selon la revendication 3, dans laquelle le sucre est le saccharose ou le dextrose.

5. Composition lyophilisée selon l'une quelconque des revendications 1 à 4, dans laquelle le tensio-actif à base d'amine est la triéthylamine ou la triéthanolamine.

6. Composition lyophilisée selon l'une quelconque des revendications 1 à 5, comprenant en outre une quantité immunologiquement efficace d'un antigène ou de plusieurs antigènes.

7. Composition de vaccins comprenant une quantité d'une composition lyophilisée selon l'une quelconque des revendications 1 à 6 et une quantité immunologiquement efficace d'un antigène ou de plusieurs antigènes.

8. Composition de vaccins selon la revendication 7, dans laquelle le ou les antigènes sont dérivés ou produits par une bactérie, un virus, un parasite, une cellule cancéreuse ou un allergène.

9. Composition de vaccins selon la revendication 7, dans laquelle l'antigène est un antigène de *Chlamydia,* d*'Haemophilus influenzae* non sérotypable, d'*Helicobacter pylori,* de *Moraxella catarrhalis,* de *Neisseria gonorrhoeae,* de *Neisseria meningitidis,* de *Salmonella typhi,* de *Streptococcus pneumoniae,* de streptocoques du Groupe A, de streptocoques du Groupe B, du virus Herpes simplex, du virus de l'immunodéficience humaine, du papillomavirus humain, du virus de la grippe, du virus de la rougeole, du virus parainfluenza, du virus respiratoire syncytial, du rotavirus ou du virus Norwalk.

10. Composition de vaccins selon l'une quelconque des revendications 7 à 9, dans laquelle la quantité immunologiquement efficace d'un antigène ou de plusieurs antigènes est de 1 µg à 5 mg.

11. Composition de vaccins selon l'une quelconque des revendications 7 à 9, dans laquelle la quantité efficace de 3-O-désacyl-4'-lipide monophosphoryle A est de 1 µg à 1 mg.

12. Composition de vaccins selon l'une quelconque des revendications 7 à 11, dans laquelle l'antigène est un conjugué composé de polysaccharide capsulaire de Streptococcus pneumoniae lié par covalence à une protéine.

13. Composition aqueuse comprenant une composition lyophilisée selon l'une quelconque des revendications 1 à 6, reconstituée avec un diluant aqueux à une quantité jusqu'à 210 mg de composition lyophilisée par ml de diluant aqueux, ladite composition aqueuse sous forme d'une suspension colloïdale aqueuse ayant une transmission de la lumière supérieure ou égale à 88% par spectrophotométrie.

14. Composition aqueuse selon la revendication 13, comprenant, par ml de diluant aqueux, jusqu'à 2,5 mg de 3-O-désacyl-4'-lipide monophosphoryle A, 10 à 200 mg de sucre et 0 à 6 mg d'un tensio-actif à base d'amine ajouté facultativement.

15. Composition aqueuse selon la revendication 13, comprenant, par ml de diluant aqueux, de 0,5 à 2,5 mg de 3-O-désacyl-4'-lipide monophosphoryle A, de 20 à 150 mg de sucre et de 0 à 3 mg d'un tensio-actif à base d'amine ajouté facultativement.

16. Composition aqueuse selon l'une quelconque des revendications 13 à 15, dans laquelle ledit diluant aqueux comprend l'eau ou une solution saline isotonique.

17. Composition aqueuse selon l'une quelconque des revendications 13 à 16, dans laquelle ledit diluant aqueux comprend en outre un antigène ou plusieurs antigènes, un phosphate d'aluminium ou autre adjuvant, ou un vecteur pharmaceutiquement acceptable.

18. Composition de vaccins comprenant une quantité d'une composition aqueuse selon l'une quelconque des revendications 13 à 17 et une quantité immunologiquement efficace d'un antigène ou de plusieurs antigènes.

19. Composition de vaccins selon la revendication 18, dans laquelle le ou les antigènes sont dérivés ou produits par une bactérie, un virus, un parasite, une cellule cancéreuse ou un allergène.

20. Composition de vaccins selon la revendication 18 ou la revendication 19, dans laquelle l'antigène est un conjugué composé de polysaccharide capsulaire de Streptococcus pneumoniae lié par covalence à une protéine.

21. Composition de vaccins selon l'une quelconque des revendications 18 à 20, dans laquelle l'antigène est un antigène de *Chlamydia, d'Haemophilus influenzae* non sérotypable, d'*Helicobacter pylori,* de *Moraxella catarrhalis,* de *Neisseria gonorrhoeae,* de *Neisseria meningitidis,* de *Salmonella typhi,* de *Streptococcus pneumoniae,* de streptocoques du Groupe A, de streptocoques du Groupe B, du virus Herpes simplex, du virus de l'immunodéficience humaine, du papillomavirus humain, du virus de la grippe, du virus de la rougeole, du virus parainfluenza, du virus respiratoire syncytial, du rotavirus ou du virus Norwalk.

22. Composition de vaccins selon l'une quelconque des revendications 18 à 21, dans laquelle la quantité efficace d'antigène est de 1 µg à 5 mg.

23. Composition de vaccins selon l'une quelconque des revendications 18 à 21, dans laquelle la quantité efficace de 3-O-désacyl-4'-lipide monophosphoryle A est de 1 µg à 1 mg.

24. Procédé pour la préparation d'une composition lyophilisée selon les revendications 1 à 6, comprenant:
a. la suspension du 3-O-désacyl-4'-lipide monophosphoryle A à la quantité jusqu'à 5 mg/ml et un tensio-actif à base d'amine à la quantité de 0 à 6 mg/ml dans un diluant aqueux;
b. la formation d'une suspension colloïdale aqueuse ayant une transmission de la lumière supérieure ou égale à 88%, par spectrophotométrie;
c. l'ajout de sucre à raison de 10 à 200 mg/ml, avant ou après la formation de la suspension colloïdale aqueuse;
d. la lyophilisation de la suspension colloïdale aqueuse contenant le sucre; et
e. la récupération d'une composition lyophilisée.

25. Procédé pour la préparation d'une composition lyophilisée selon les revendications 1 à 6, comprenant:
a. le chauffage de lipopolysaccharide de bactéries gram-négatives *Salmonella minnesota* R595 dans un acide minéral de force modérée pendant un temps suffisant pour obtenir un dérivé monophosphoryle;
b. la dissolution du dérivé monophosphoryle dans un solvant organique et séchage;
c. le traitement du dérivé monophosphoryle avec un alcali doux pour éliminer une chaîne d'acide gras labile aux bases en position 3, pour donner le 3-O-désacyl-4'-lipide monophosphoryle A;
d. la purification du 3-O-désacyl-4'-lipide monophosphoryle A par chromatographie liquide et la récupération du lipide monophosphoryle A;
e. la suspension du 3-O-désacyl-4'-lipide monophosphoryle A à la quantité jusqu'à 5 mg/ml et d'un tensio-actif à base d'amine à la quantité de 0 à 6 mg/ml dans un diluant aqueux;
f. la formation d'une suspension colloïdale aqueuse ayant une transmission de la lumière supérieure ou égale à 88%, par spectrophotométrie;
g. l'ajout de sucre à raison de 10 à 200 mg/ml, avant ou après la formation de la suspension colloïdale aqueuse;
h. la lyophilisation de la suspension colloïdale aqueuse contenant le sucre; et
i. la récupération d'une composition lyophilisée.

26. Procédé pour la préparation d'une suspension colloïdale aqueuse selon les revendications 13 à 17, contenant du 3-O-désacyl-4'-lipide monophosphoryle A capable d'être congelée et décongelée, comprenant:
a. la suspension du lipide monophosphoryle A à la quantité jusqu'à 5 mg/ml et d'un tensio-actif à base d'amine à la quantité de 0 à 6 mg/ml dans un diluant aqueux;
b. la formation d'une suspension colloïdale aqueuse ayant une transmission de la lumière supérieure ou égale à 88%, par spectrophotométrie;
c. l'ajout de sucre à raison de 10 à 200 mg/ml, avant ou après la formation de la suspension colloïdale aqueuse;
d. la congélation de la suspension colloïdale aqueuse contenant le sucre; et
e. la décongélation et la récupération de la suspension colloïdale aqueuse.

27. Procédé pour la préparation d'une suspension colloïdale aqueuse selon les revendications 13 à 17, contenant du 3-O-dësacyl-4'-lipide monophosphoryle A capable d'être lyophilisée et remise en suspension, comprenant:
a. la suspension du 3-O-désacyl-4'-lipide monophosphoryle A à la quantité jusqu'à environ 5 mg/ml et d'un tensio-actif à base d'amine à la quantité de 0 à environ 6 mg/ml dans un diluant aqueux;
b. la formation d'une suspension colloïdale aqueuse ayant une transmission de la lumière supérieure ou égale à 88%, par spectrophotométrie;
c. l'ajout de sucre à raison de 10 à 200 mg/ml, avant ou après la formation de la suspension colloïdale aqueuse;
d. la lyophilisation de la suspension colloïdale aqueuse contenant le sucre; et
e. la remise en suspension et la reformation de la suspension colloïdale aqueuse.

28. Procédé selon la revendication 26 ou la revendication 27, dans lequel ladite suspension colloïdale aqueuse lyophilisée est combinée avec un diluant aqueux contenant en outre un antigène ou plusieurs antigènes, un adjuvant phosphate d'aluminium ou un conservateur, vecteur ou excipient pharmaceutiquement acceptable.

29. Suspension colloïdale aqueuse préparée selon le procédé de l'une quelconque des revendications 26 à 28.

30. Utilisation d'une composition lyophilisée selon l'une quelconque des revendications 1 à 6, pour la préparation d'un vaccin.

31. Utilisation d'une composition lyophilisée selon l'une quelconque des revendications 1 à 6, pour la préparation d'une composition aqueuse selon l'une quelconque des revendications 13 à 17.

32. Utilisation d'une composition aqueuse selon l'une quelconque des revendications 13 à 17, pour la préparation d'un vaccin.

33. Utilisation d'une composition de vaccins selon l'une quelconque des revendications 7 à 12 ou 18 à 23 pour la préparation d'un médicament pour l'immunisation d'un vertébré.
